# EUROPÄISCHE PATENTSCHRIFT

(11) **EP 2 672 938 B1**
(45) Veröffentlichungstag und Bekanntmachung des Hinweises auf die Patenterteilung: **02.05.2018**
(21) Anmeldenummer: 12706453.3
(22) Anmeldetag: 09.02.2012
(51) Int. Cl.: A61F 2/90, A61F 5/08, A61F 5/56, A61M 29/02

(54) **STENT ZUM SCHIENEN EINES NASENGANGES**
STENT FOR SPLINTING A NASAL PASSAGE
STENT DESTINÉ À SERVIR D'ATTELLE D'UNE VOIE NASALE

(30) Priorität: 09.02.2011 DE 102011010754
(43) Veröffentlichungstag der Anmeldung: 18.12.2013
(73) Patentinhaber: Düring, Klaus, 50226 Frechen (DE)
(72) Erfinder: RENNER, Peter, 50129 Bergheim (DE); DÜRING, Klaus, 50226 Frechen (DE); PFEFFER, Joachim, Georg, 52070 Aachen (DE); DLAIKAN-CAMPOS, Nasib, 52134 Herzogenrath (DE)
(74) Vertreter: Schüssler, Andrea
(86) Internationale Anmeldenummer: PCT/EP2012/000589
(87) Internationale Veröffentlichungsnummer: WO 2012/107229

(56) Entgegenhaltungen:
- WO-A2-03/092765
- WO-A2-2007/065408
- DE-A1- 2 141 252

## Beschreibung

Die vorliegende Erfindung betrifft einen Stent zum Schienen eines Nasenganges. Unter dem Handelsnamen "Schnarchzapfen" werden Kunststoffschienen zum Schienen der Nasenöffnungen angeboten. Diese Kunststoffschienen bestehen jeweils aus mehreren Kunststoffringen, die über Längsstreben miteinander verbunden sind. An einem Ende sind zwei derartige Kunststoffschienen mittels eines Kunststoffbügels miteinander verbunden, so dass jeweils zwei Kunststoffschienen in die beiden Löcher einer Nase gesteckt werden können. An dem mit dem Bügel verbundenen Ende ist der Durchmesser der Kunststoffschiene größer als an dem vom Bügel entfernten Ende. Diese Schienen werden somit mit dem schmäleren Ende voran in die Nase gesteckt. Diese Schienen sind etwa 10 mm lang und stellen sicher, dass die Nase im Bereich ihrer Öffnung geöffnet ist.

Unter dem Markenname Nasaline® werden Nasendilatatoren angeboten, die die Nasenflügel erweitern und damit die Mundatmung verringern sollen. Diese Nasendilatatoren sind kurze Kunststoffbuchsen, die über einen Kunststoffbügel lose miteinander verbunden sind, so dass sie in ihrem Abstand verstellbar sind.

Diese Produkte "Schnarchzapfen" und Nasaline® sind jeweils buchsen- bzw. rohrförmige Körper, die die Nasenflügel offenhalten sollen.

Anstelle von derartigen Buchsenelementen gibt es auch Flügel, mit welchen die Nasenflügel auseinandergedrückt werden. Ein solches Element mit zwei Abstandsflügeln, die über einen steifen Nasenbügel aus Titan in einem vorbestimmten Abstand gehalten werden, ist unter dem Handelsnamen Nasanita Nasenschmetterling® bekannt. Ein auf einem ähnlichen Wirkprinzip beruhendes Element zum Offenhalten der Nasenflügel wird unter dem Handelsnamen Nozovent® vertrieben.

Die oben erläuterten Vorrichtungen dienen zum Offenhalten der Nasenöffnungen.

Von der Firma MBM ScienceBridge GmbH, Göttingen, Deutschland wurde eine Nasopharyngeale Schiene entwickelt. Diese Schiene besteht aus zwei Kunststoffschienen, die jeweils muldenförmig geformt sind, wobei die muldenförmigen Schienen an einem Ende mit einem langgestreckten Bügel miteinander verbunden sind. Eine jede Schiene kann in ein Nasenloch eingeführt werden. Die Schienen sind so geformt, dass sie sich durch die Nase, vorbei an der Nasenscheidewand einführen lassen und den oberen Rachen durch seine Form offenhalten. Das Gaumensegel wird daran gehindert, den Rachen zu schließen. Dadurch soll eine kontinuierliche Atmung gewährleistet sein. Bei der Schnarchtherapie werden dem Patienten einmalig per Endoskopie die Schienen angepasst. Dies ist notwendig, um zu gewährleisten, dass die Länge der Schiene exakt eingestellt ist.

Aus der WO 2007/065408 A2 geht ein Apnoe-Stent hervor, der zur Schienung und/oder Offenhaltung des Atemwegs im Rachenraum dient. Dieser Apnoe-Stent besteht aus einem komprimierbaren und selbst-expandierenden Stent, der zumindest einen aufgeweiteten Bereich aufweist. Dieser Stent ist dreiphasig ausgebildet. Eine distale Phase des Stents bildet einen aktiven Teil des Apnoe-Stents aus. Diese distale Phase ist rohrförmig ausgebildet und derart aufweitbar, dass der Atemweg offengehalten wird. Eine proximale Phase des Stents ist zur Fixierung des Stents im Nasenbereich vorgesehen. Die proximale Phase ist trichterförmig ausgebildet, wobei sie sich von einem proximalen zu einem distalen Ende hin aufweitet. Die distale und die proximale Phase sind als Geflecht ausgebildet, bei dem sich Drähte bzw. Fasern bzw. Fäden kreuzen. Jeder einzelne in Richtung zum distalen Ende des Stents verlaufende Draht wird am distalen Ende der distalen Phase zurück zum proximalen Ende des Stents geführt. Die hierdurch erzeugten Biegungen werden als runde Enden bezeichnet. Eine Übergangsphase des Stents ist zum Verbinden der proximalen Phase mit der distalen Phase vorgesehen. Die Übergangsphase ist durch Verzwirbelung der Drähte des Stents zu verzwirbelten Strängen ausgebildet. Hierdurch weist der Übergangsbereich in Radialrichtung eine hohe Flexibilität auf, so dass innerhalb einer kurzen Längserstreckung eine starke radiale Aufweitung von der proximalen zur distalen Phase bewirkt wird.

Aus der DE 20 2009 010 388 U1 bzw. der PCT/EP 2010/004687 geht eine Fixiervorrichtung zur Fixierung eines solchen Apnoe-Stents im Atemweg hervor, wobei die Fixiereinrichtung zwei Klemmelemente aufweist, zwischen welchen das proximale Ende des Apnoe-Stents fixierbar ist. Weiterhin ist hierin ein Einführschlauch zum Einführen des Apnoe-Stents beschrieben, der am distalen Ende gebogen ist, um das Einführen in einen Atemweg zu erleichtern. Der Apnoe-Stent weist an seinem proximalen Ende ein Verbindungs- oder Kopplungselement auf, so dass eine Einführstange an das proximale Ende des Apnoe-Stents fixiert werden kann. Mit Hilfe der Einführstange kann der Stent in den Einführschlauch 6 eingeführt und komprimiert werden, indem die Einführstange durch den Einführschlauch geschoben wird, so dass der Apnoe-Stent von der Einführstange in den Einführschlauch gezogen wird.

Aus der US 2009/0010991 A1 geht ein expandierbarer Nasen-Stent hervor, der dauerhaft in die Nasenöffnung bzw. den Nasengang eingesetzt wird. Dieser Stent ist aus einem Kunststoff oder Stahlgitter ausgebildet und mit einem Filterelement versehen, das im proximalen Ende des Stents angeordnet ist. Der Stent kann mit einem Wirkstoff beschichtet sein.

Aus der US 2010/0211181 A1 und der US 5,336,163 gehen weitere Nasen-Stents hervor, die als Filter ausgebildet sind, um die eingeatmete Luft zu filtern.

Die US 2010/0106255 A1 offenbart einen selbst-expandierenden Stent, der mit einem Ende in den Stirnhöhlen verankert werden kann.

Aus der DE 2141252 A1 geht ein korbartig ausgebildeter Naseneinsatz zum Abstützen der Nasenhöhle hervor, der in etwa eine birnenförmige Form aufweist und aus einem elastisch verformbaren Kunststoff ausgebildet ist.

Der vorliegenden Erfindung liegt die Aufgabe zugrunde, einen Stent zum Schienen eines Nasenganges zu schaffen, der nicht an einen jeden Nasengang individuell angepasst werden muss, der einfach einführbar und angenehm zu benutzen ist und einen Nasengang vollständig und zuverlässig offenhält.

Die Aufgabe wird durch einen Stent mit dem Merkmal des Anspruchs 1 gelöst. Vorteilhafte Ausgestaltungen sind in den Unteransprüchen angegeben.

Erfindungsgemäß ist ein Stent zum Schienen eines Nasenganges vorgesehen, wobei der Stent aus einem geflochtenen, rohrförmigen Stützkörper ausgebildet ist. Der erfindungsgemäße Stent zeichnet sich dadurch aus, dass der Stützkörper im Wesentlichen zylinderförmig ausgebildet ist und an seinem proximalen Ende einen aufgeweiteten Abschnitt aufweist, der etwa kugelförmig aufgeweitet ist, wobei der Stützkörper im unbelasteten Zustand einen Durchmesser von 4 bis 20 mm und eine Länge von 25 bis 100 mm aufweist.

Der erfindungsgemäße Stent zum Schienen eines Nasenganges besteht aus einem geflochtenen, schlauchförmigen Stützkörper und optional einem Fixierabschnitt. Der Stützkörper weist im unbelasteten Zustand einen Durchmesser von mindestens 4 mm und eine Länge im Bereich von 25 mm bis 120 mm und insbesondere von 25 mm bis 100 mm auf.

Am proximalen Ende des Stents kann ein Fixierabschnitt angeordnet sein, der zum Fixieren des Stents in einem Nasengang eines Benutzers ausgebildet ist, wobei der Fixierabschnitt sich beim Fixieren aus dem Nasenloch des Benutzers heraus erstreckt und außerhalb der Nase fixierbar ist.

Der geflochtene Stützkörper ist elastisch verformbar und schmiegt sich an die Innenfläche des Nasenganges an. Der vom rohrförmigen Stützkörper auf den Nasengang erzeugte Druck verteilt sich gleichmäßig, wodurch der Stützkörper in dem empfindlichen Nasengang keine Schmerzen oder unangenehme Gefühle verursacht. Da der Stützkörper geflochten ist, schmiegt er sich an unterschiedliche Formen des Nasenganges an und es ist nicht notwendig, den Stent an den Nasengang eines bestimmten Benutzers individuell anzupassen. Mit einigen wenigen Standardgrößen des Stents, die sich vor allem in der Länge und im Durchmesser des Stützkörpers unterscheiden, kann für fast jede Person ein geeigneter Stent vorgesehen werden. Der Stützkörper kann in Durchmessern von 4 bis 20 mm ausgebildet sein, wobei sich die Standardgrößen vorzugsweise im Durchmesser unterschieden und jeweils 1 mm abgestuft sind. Die Länge des Stützkörpers liegt im Bereich von 25 mm bis 120 mm und insbesondere von 25 mm bis 100 mm. Vorzugsweise sind die Standardgrößen in 5 mm-Stufen in der Länge ausgebildet.

Die bevorzugte Länge des Stützkörpers liegt im Bereich von 30 mm bis 50 mm, da dies die üblichen Längen der Nasengänge im Bereich der vorderen Nasenmuscheln bei durchschnittlichen Körpergrößen sind.

Es hat sich gezeigt, dass bei den meisten Nutzern ein Stützkörper mit einer Länge von etwa 60 mm mit seinem distalen Ende an einem Bereich der Nasengänge und Nasenmuscheln liegt, die sehr empfindlich sind. Deshalb ist es meistens zweckmäßig, den Stützkörper entweder kürzer auszubilden, so dass er mit diesem empfindlichen Bereich nicht in Kontakt gelangt, oder länger auszubilden, so dass er sich durch diesen empfindlichen Bereich hindurch erstreckt. Daher sind einerseits Stützkörper mit einer Länge von maximal 40 mm bis etwa 45 bzw. 50 mm oder Stützkörper mit einer Länge von zumindest 70 mm bis 80 mm bzw. 100 mm bevorzugt.

Die Funktion des Fixierabschnittes liegt darin, dass er verhindert, dass der Stützkörper durch den Nasengang hindurch in Richtung zum Rachen rutschen kann. Der Fixierabschnitt ist deshalb mit dem Stützkörper verbunden und erstreckt sich bis außerhalb der Nase. Hier kann der Fixierabschnitt fixiert werden.

Der Fixierabschnitt kann einteilig mit dem Stützkörper als geflochtener, schlauchförmiger, sich zum proximalen Ende hin verjüngender Abschnitt ausgebildet sein. Der Fixierabschnitt kann jedoch auch aus einem beliebigen anderen Material bestehen, das vorzugsweise flexibel ist.

Am proximalen Ende des Fixierabschnittes ist ein Kupplungselement angeordnet, das mit einem Gegenkupplungselement einer Einführstange oder eines Haltelements koppelbar ist. Das Gegenkupplungselement ist an einem Ende der Einführstange angeordnet. Mit der an den Stent gekoppelten Einführstange kann der Stent in einen Einführschlauch eingeführt und komprimiert werden. Hierzu wird die Einführstange durch den Einführschlauch geschoben, so dass der Stent von der Einführstange in den Einführschlauch gezogen wird.

Vorzugsweise weist das Halteelement zwei Gegenkupplungselemente zum Koppeln zweier proximaler Enden zweier Stents an das Halteelement auf. Hierdurch bildet das Halteelement einen lösbaren Bügel zwischen den zwei Stents, der sicher verhindert, dass die beiden Stents zu tief in Richtung des Rachens verschoben werden.

Das Halteelement kann auch eine Klammer sein, die einen Fixierabschnitt eines Stents oder zwei Fixierabschnitte zweier Stents durch eine Klemmung fixiert. Die Klemmung kann unabhängig vom Kupplungselement zum Koppeln des Stents an eine Einführstange oder aber durch Klemmung des Kupplungselements im Halteelement erfolgen.

Der Stent kann aus Drähten, Fasern und/oder Fäden, die sich kreuzen, geflochten sein. Im Folgenden wird lediglich der Begriff Draht verwendet, wobei hiermit auch Fasern oder Fäden gemeint sind, sofern der Draht nicht näher definiert ist.

Jeder einzelne in Richtung zum distalen Ende des Stents verlaufende Draht wird am distalen Ende des Stützkörpers zurück zum proximalen Ende des Stents geführt. Die hierdurch erzeugten Biegungen werden als runde Enden bezeichnet. Diese weisen im unbelasteten Zustand des Stents einen Durchmesser im Bereich von ca. 0,5 bis 2 mm auf. Auf diese Weise wird ein distales Ende bereitgestellt, an dem keine einzelnen Drahtenden freiliegen oder mit einem weiteren Element miteinander verbunden werden müssen. Die Verbindung des Drahtes bzw. der Drähte des Stents kann am proximalen Ende des Stents z.B. durch Crimpen oder Verkleben erfolgen, so dass ein Fixierabschnitt ausgebildet wird Alternativ können die Drähte auch ohne die Ausbildung eines Fixierabschnitts zusammengefasst werden, z.B. durch einen Kunststofftropfen, einen Ring oder indem sie nach innen zurückgeführt werden in den Stützkörper. Durch die runden Enden werden Verletzungen der Atemwege durch das distale Ende des Stents vermieden.

Der Draht, aus dem der Stützkörper geflochten ist, ist insbesondere ein Metalldraht, vorzugsweise ein Nitinoldraht oder ein Stahldraht.

Vorzugsweise ist das distale Ende des Stützkörpers, am meisten bevorzugt nur die runden Enden, gegenüber dem übrigen Bereich des Stützkörpers etwas verjüngt ausgebildet. Hierdurch wird verhindert, dass sich der Stent mit seiner Endkante in den Nasengang eindrückt. Dies kann unangenehm sein.

Der Stützkörper kann an seinem proximalen Ende einen aufgeweiteten Abschnitt aufweisen. Dieser aufgeweitete Abschnitt ist vorzugsweise etwa kugelförmig aufgeweitet. Die Kugel weist einen Durchmesser von etwa 10 bis 20 mm, vorzugsweise 12 bzw. 13 bis 15 bzw. 17 mm auf. Dieser aufgeweitete Abschnitt des Stützkörpers wird bei Gebrauch im Bereich unmittelbar hinter den Nasenöffnungen angeordnet, um die Nasenflügel gegen einen Kollaps abzustützen. Zusätzlich fixiert der aufgeweitete Abschnitt den Stent in der Nase, so dass insbesondere bei einem Stent mit aufgeweitetem Abschnitt der oben erläuterte Fixierabschnitt entfallen kann. Ein solcher Stent ist besonders für sportliche Tätigkeiten geeignet, bei welchen die Nasengänge offen gehalten werden sollen. Ein an der Nase vorstehender Fixierabschnitt ist bei solchen sportlichen Tätigkeiten nicht erwünscht.

Bei einem Stent ohne Fixierabschnitt ist es zweckmäßig ein Entfernungswerkzeug vorzusehen, mit dem der Stent aus einem Nasengang entfernt werden kann. Das Entfernungswerkzeug ist an den Stent koppelbar, bspw, indem es ein Element zum Einhaken in das Geflecht des Stents oder ein Element zum Greifen des Geflechts aufweist. Zum Koppeln an das Entfernungswerkzeug genügt alleine der geflochtene Stützkörper. Hierzu muss am Stent kein weiterer Bestandteil vorgesehen werden.

Die Erfindung wird nachfolgend beispielhaft anhand der Zeichnungen näher erläutert. Die Zeichnungen zeigen in:
- Fig. 1: einen nicht-erfindungsgemäßen Stent nach einem Ausführungsbeispiel schematisch in einer Seitenansicht,
- Fig. 2: einen nicht-erfindungsgemäßen Stent nach einem weiteren Ausführungsbeispiel schematisch in einer Seitenansicht,
- Fig. 3: einen erfindungsgemäßen Stent schematisch in einer Seitenansicht,
- Fig. 4: einen nicht-erfindungsgemäßen Stent nach einem weiteren Ausführungsbeispiel schematisch in einer Seitenansicht,
- Fig. 5a, 5a: jeweils ein Haltelement in der Draufsicht,
- Fig. 6: ein weiteres Haltelement in der Draufsicht,
- Fig. 7a, 7b: ein Halteelement in der Draufsicht und in einer Seitenansicht,
- Fig. 8: einen Abschnitt einer Einführstange in einer Seitenansicht,
- Fig. 9: einen Abschnitt eines Einführschlauches in einer Schnittansicht,
- Fig. 10: schematisch die Nasenhöhle in einer Schnittdarstellung,
- Fig. 11a und 11b: schematisch grob vereinfacht ein weiteres Halteelement in der Seitenansicht,
- Fig. 12a und 12b: schematisch grob vereinfacht ein weiteres Halteelement in der Seitenansicht,
- Fig. 13a und 13b: schematisch grob vereinfacht ein weiteres Halteelement in der Seitenansicht,
- Fig. 14a bis 14c: schematisch grob vereinfacht ein weiteres Halteelement in der Seitenansicht,
- Fig. 15a, b bis 17a, b: jeweils einen proximalen Endbereich eines Stents ohne Fixierabschnitt in einer Vorderansicht (Fig. a) und in einer Seitenansicht (Fig. b), und
- Fig. 18a, 18b, 19a, 19b, 19c, 20a, 20b, 20c: jeweils ein Entfernungswerkzeug zum Entfernen eines Stents au seiner Nase in unterschiedlichen Greifzuständen.

Ein erfindungsgemäßer Stent 1 zum Schienen eines Nasenganges umfasst einen Stützkörper 2 und einen Fixierabschnitt 3.

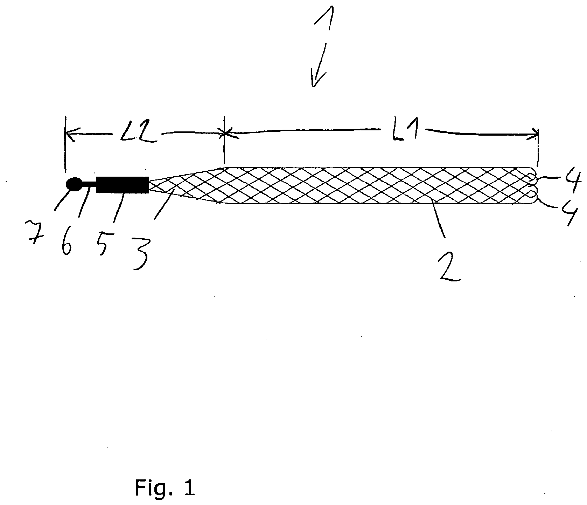

Fig. 1 zeigt ein erstes Ausführungsbeispiel eines nicht-erfindungsgemäßen Stents 1, bei dem der Stützkörper 2 rohrförmig mit konstantem Durchmesser ausgebildet ist und der Fixierabschnitt 3 sich von seinem distalen Ende in Richtung zu seinem proximalen Ende konisch verjüngt. Sowohl der Stützkörper 2 als auch der Fixierabschnitt 3 sind integral aus einem Drahtgeflecht ausgebildet. Jeder einzelne, in Richtung zum distalen Ende des Stents 1 verlaufende Draht wird am distalen Ende des Stützkörpers 2 zurück zum proximalen Ende des Stents 1 geführt. Die hierdurch erzeugten Biegungen werden als runde Enden 4 bezeichnet. Diese weisen im unbelasteten Zustand des Stents einen Durchmesser im Bereich von ca. 0,5 bis 2 mm auf. Auf diese Weise wird ein distales Ende bereitgestellt, an dem keine einzelnen Drahtenden freiliegen oder mit einem weiteren Element miteinander verbunden werden müssen. Durch die runden Enden werden Verletzungen des Nasenganges durch das distale Ende des Stents vermieden.

Der Stent 1 kann aus einem einzigen Draht oder aus einer Vielzahl von Drähten geflochten sein. Die Enden des Drahtes bzw. der Drähte sind am proximalen Ende des Stents bzw. am proximalen Ende des Fixierabschnitts 3 mit einem stiftförmigen bzw. zylinderförmigen Körper 5 zusammengefasst. Im vorliegenden Ausführungsbeispiel ist der stiftförmige Körper 5 ein um die Enden der Drähte gecrimpter Körper 5. Man kann ihn deshalb auch als Crimpkörper 5 bezeichnen. Am distalen Ende des Crimpkörpers 5 ist ein dünner Stift 6 angeformt. Am vom Crimpkörper 5 entfernten Ende des Stiftes 6 ist eine Kugelkupplung 7 ausgebildet. Mit der Kugelkupplung 7 kann der Stent an ein anderes Element gekoppelt werden, das ein entsprechendes Gegenkupplungselement aufweist.

Der Stent ist aus einem elastischen Draht mit einem Durchmesser von 0,001 mm bis 2 mm, insbesondere mit einem Durchmesser von 0,05 mm bis 0,5 mm und vorzugsweise mit einem Durchmesser von 0,07 mm bis 0,2 mm geflochten. Der Draht ist insbesondere ein Metalldraht und vorzugsweise ein Nitinoldraht oder Stahldraht.

Die Länge L2 des Stützkörpers 2 beträgt 25 mm bis 120 mm und insbesondere 25 mm bis 100 mm. Vorzugsweise liegt die Länge L2 des Stützkörpers im Bereich von 30 mm bis 50 mm oder von 60 mm bis 120 mm bzw. von 30 mm bis 50 mm oder von 70 mm bis 100 mm. Bei der längeren Ausführung ist am meisten bevorzugt ein Bereich von 70 bis 100 mm.

Der Durchmesser des Stützkörpers 2 liegt im Bereich von 4 mm bis 20 mm. Vorzugsweise weist der Stützkörper einen Durchmesser von zumindest 5 mm und insbesondere von zumindest 6 mm auf.

Der Fixierabschnitt 3 weist eine Länge L2 von etwa 10 bis 25 mm auf. Mit dem Fixierabschnitt kann der Stent derart am Körper des Benutzers fixiert werden, dass der Stent nicht aus dem Nasengang des Benutzers in Richtung zum Rachen verschoben werden kann. Hierzu ist es notwendig, dass der Fixierabschnitt sich durch die Nasenöffnung nach außen erstreckt, um dann dort mit einem entsprechenden Fixierelement fixiert zu werden. Ein solches Fixierelement kann beispielsweise ein Pflaster sein, mit dem der Fixierabschnitt 3 im Bereich unterhalb der Nase des Benutzers festgeklebt wird. Bevorzugt wird jedoch ein Haltelement 8, das sich zumindest in eine Richtung soweit erstreckt, dass es nicht durch eine Nasenöffnung in den Nasengang eingeführt werden kann. Alternativ kann das Halteelement 8 so ausgebildet sein, dass es mit zwei Stents 1 koppelbar ist, so dass es sich wie ein Bügel zwischen beiden Stents erstreckt und so ein zu tiefes Einziehen des Stents in den Nasengang verhindert. Ein solches Halteelement 8, das unten näher erläutert wird, wird vorzugsweise an das Kupplungselement des Stents, das im vorliegenden Ausführungsbeispiel als Kugelkupplung 7 ausgebildet ist, gekoppelt. Im Rahmen der Erfindung ist es jedoch auch möglich, als Haltelement eine Klemme vorzusehen, die an den Fixierabschnitt 3 eines Stents 1 oder an die Fixierabschnitte 3 zweier Stents 1 geklemmt wird.

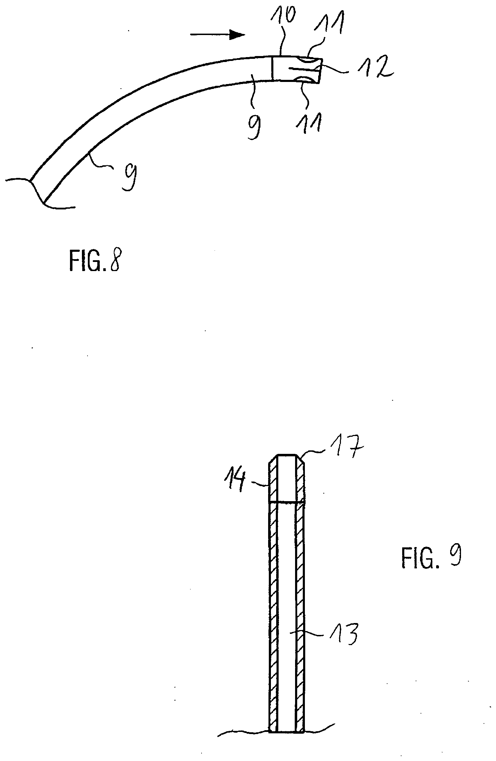

Eine Einführstange 9, die an das Kupplungselement 7 gekoppelt werden kann, ist in Fig. 8 gezeigt. Die Einführstange 9 ist aus Kunststoff ausgebildet. Am distalen Ende der Einführstange 9 ist einstückig eine rohrförmige Buchse 10 angeformt. Die Buchse weist zwei diametral einander gegenüberliegend angeordnete kreisförmige Durchgangsöffnungen 11 auf. In Längsrichtung der Einführstange 9 kann im Bereich der beiden kreisförmigen Durchgangsöffnungen 11 ein Schlitz 12 ausgebildet sein. Der Schlitz 12 erstreckt sich bis zum distalen Ende der Buchse 10. Die Buchse 10 kann direkt am distalen Ende der Einführstange 9 ausgebildet oder separat aus einem anderen Material hergestellt und kraftschlüssig mit der Einführstange 9 verbunden, zum Beispiel verklebt, sein. Die Kugelkupplung 7 des Stents 1 ist zwischen den beiden Durchgangsöffnungen 11 der Buchse 10 der Einführstange 9 aufnehmbar. Auf diese Weise wird eine lösbare Verbindung zwischen dem Stent 1 und der Einführstange 9 bereitgestellt. Der in der Buchse 10 ausgebildete Längsschlitz 12 erleichtert das Einführen und Herausziehen der Kugelkupplung 7 in bzw. aus der Buchse 10. Über das Verhältnis des Durchmessers der Kugelkupplung 7 zum Innendurchmesser der Buchse 10 sowie über die Härte des Materials der Buchse 10 und die Länge des Schlitzes 12 ist die Verbindungs- bzw. Haltekraft der Verbindung zwischen der Kugelkupplung 7 und der Buchse 10 einstellbar. Die Kugelkupplung 7 ist frei drehbar in der Buchse 10 gelagert.

Die Einführstange kann auch als Einführrohr (nicht dargestellt) ausgebildet werden, in das an einem Ende die beiden kreisförmigen Durchgangsöffnungen und optional ein Schlitz - ähnlich wie bei der Buchse 10 - eingearbeitet sind. Dadurch entfällt die separate Herstellung der rohrförmigen Buchse.

Der Durchmesser der Einführstange 9 und des Crimpkörpers 5 sind so bemessen, dass die Einführstange 9 zusammen mit dem Stent 1 in einen Einführschlauch 13 eingeführt werden können (Fig. 9). Der Einführschlauch 13 ist aus einem ausreichend harten, geeigneten Kunststoff, wie zum Beispiel PEBAX mit einer Shore-Härte von 50 Shore D bis 80 Shore D ausgebildet, beispielsweise PEBAX 7233 (Shorehärte 69D), PEBAX 6333 (Shorehärte 64D) oder PEBAX 55 (Shorehärte 54D). Am distalen Ende des Einführschlauchs 13 kann eine Einführspitze 14 einstückig angeformt sein. Die Einführspitze entspricht sowohl im Innen- als auch im Außendurchmesser dem Einführschlauch 13 und ist mit diesem bündig ausgebildet. Die Einführspitze 14 kann aus einem flexiblen, wesentlich weicheren Material, wie zum Beispiel PEBAX mit einer Shore-Härte von 25 Shore D bis 45 Shore D ausgebildet sein. Der Einführschlauch 13 kann auch einteilig mit der Einführspitze 14 ausgebildet sein. Ein solcher einteiliger Einführschlauch kann aus einem einheitlichen Material, wie zum Beispiel PEBAX 7233, PEBAX 6633 oder PEBAX 5533 bestehen und es ist möglich, einen solchen einteiligen Einführschlauch mit einem Extrusionsverfahren mit kontinuierlichem Materialgradienten herzustellen. Bei einem solchen Einführschlauch gibt es einen Übergangsbereich, in dem kontinuierlich der Anteil des einen Materials abnimmt und dementsprechend der Anteil des anderen Materials zunimmt. Die Materialien eines solchen Schlauches sind zum Beispiel PEBAX 7233 im Bereich des Hauptkörpers und PEBAX 3533 im Bereich der Einführspitze 14, so dass die Einführspitze weicher als der Hauptkörper ausgebildet ist. Bezüglich der Materialauswahl gelten die obigen Ausführungen zum zweiteiligen Einführschlauch gleichermaßen.

Der Einführschlauch kann auch mehrschichtig ausgebildet sein, wobei die innere Schicht vorzugsweise härter als die äußere Schicht ist, um innen eine glatte Oberfläche mit geringer Reibung bereitzustellen (z.B. PEBAX 7233). Hierdurch kann der Stent mit geringem Widerstand in den Einführschlauch eingeführt und wieder herausgezogen werden. Die äussere Schicht hat vorzugsweise eine Shorehärte von z.B. 60A bis 95A und ist dicker als die innenliegende Schicht, damit der Einführschlauch insgesamt in seinen Materialeigenschaften weich und flexibel ist und sich dadurch gut an den Verlauf des Nasengangs anpasst. Auf diese Weise ist der Schlauch leicht einführbar. Vorzugsweise wird die äussere Schicht aus PU 85A mit einer Schichtdicke von ca. 0,2 mm ausgeführt und die innenliegende aus PEBAX 7233 mit einer Schichtdicke von ca. 0,1 mm. Durch Anschmelzen einer kurzen Spitze aus PU 85A (ca. 2 bis 10 mm lang), die vorzugsweise atraumatisch verrundet ist, werden Komfort und Sicherheit bei der Einführung des Schlauches durch den Nasengang noch weiter gesteigert. Der Einführschlauch 13 kann geradlinig geformt sein. Er kann jedoch auch eine Krümmung aufweisen.

Der Außendurchmesser des Einführschlauchs beträgt bis zu 10 mm, bevorzugt bis zu 5 mm, um dem Benutzer eine komfortable, schmerzfreie Einführung des Stents zu ermöglichen. Der Innendurchmesser des Einführschlauchs beträgt bis zu 4 mm, um ausreichend Raum zur Aufnahme des Stents 1 bereitzustellen.

Die Einführspitze weist am außenseitigen Randbereich eine Fase 17 auf. Anstelle der Fase 17 kann die Einführspitze 14 auch mit einer Rundung ausgebildet sein. Eine solche Rundung kann beispielsweise mittels einer thermischen Umformung oder mittels Schleifen oder Laserabtragen erzeugt werden. Eine solche Rundung kann außenseitig und/oder innenseitig ausgebildet sein.

Eine solche abgerundete Spitze ist in der Herstellung aufwändiger als eine Fase. Die abgerundete Spitze ist jedoch noch sicherer beim Einführen des Stents. Zudem kann bei einer Fase das sich ergebende dünnwandige Ende verhärten, indem die Weichmacher aus dem Kunststoffmaterial durch die relativ große Oberfläche entweichen.

Der Einführschlauch kann auch eine oliven- oder tropfenförmige Verdickung an einem Ende oder beiden Enden aufweisen, um das Einführen in den Nasengang zu erleichtern. Die Olive oder der Tropfen am Ende des Einführschlauchs hat eine Länge von 2-20 mm, vorzugsweise 2-10 mm und noch mehr bevorzugt 2-5 mm. Der Durchmesser der Olive oder des Tropfens ist etwa 3,5-6,0 mm, bevorzugt 4,0-5,0 mm.

Diese geformten Einführspitzen 14 stellen atraumatische Spitzen dar, wobei die Form umso glatter und runder sein sollte, je härter das Material der Spitze ist. Eine oliven- oder tropfenförmige Ausführung weist die größte Stirnfläche auf und somit die beste Druckverteilung auf das Nasengewebe.

Zum Einführen des Stents 1 in den Einführschlauch 13 wird der Stent 1 zunächst mittels der Kugelkupplung 7 an der Buchse 10 der Einführstange 9 fixiert. Die Einführstange wird dann mit ihrem proximalen Ende an der Einführspitze 14 des Einführschlauches 13 in diesen eingeführt. Die Einführstange 9 wird durch den Einführschlauch 13 hindurchgeschoben bis der Stent 1 vollständig im Einführschlauch 13 aufgenommen ist. Hierbei kann der Stent 1 auch geringfügig am Einführschlauch etwas vorstehen. Wesentlich ist, dass der Stent 1 durch das Einführen in den Einführschlauch 13 auf einen geringen Durchmesser komprimiert ist, so dass er zusammen mit dem Einführschlauch und der Einführstange einfach in den Nasengang eingeführt werden kann. Alternativ wird der Stent 1 ohne ein Kupplungselement 7 in den Einführschlauch 13 hineingeschoben, wobei keine lösbare Verbindung mit einer Einführstange hergestellt wird. Ausführungsformen ohne Kupplungselement werden unten noch näher erläutert.

Ist diese Anordnung aus Stent 1, Einführschlauch 13 und Einführstange 9 so weit eingeführt, dass sich der Stützkörper 2 im Nasengang befindet, wird zunächst der Einführschlauch 13 über die Einführstange 9 hinweg abgezogen und dann die Einführstange vom Stent 1 gelöst. Die glatte Oberfläche des Einführschlauches 13 erleichtert erheblich das Einführen des Stents 1 in den Nasengang.

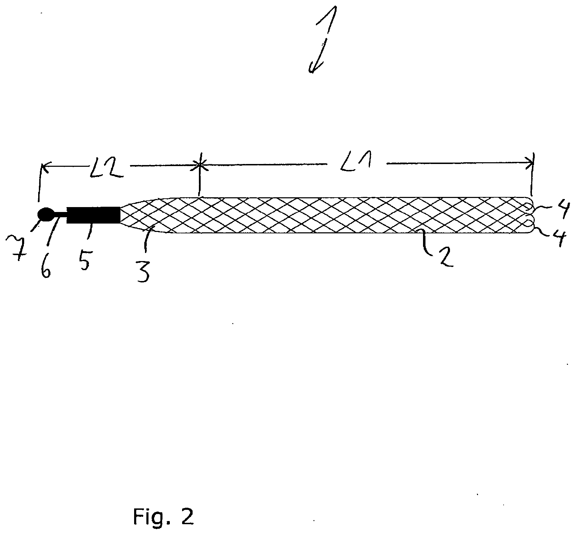

Fig. 2 zeigt ein zweites Ausführungsbeispiel des nicht-erfindungsgemäßen Stents 1, das ähnlich wie das erste Ausführungsbeispiel ausgebildet ist, weshalb gleiche Teile mit gleichen Bezugszeichen bezeichnet werden und nicht nochmals erläutert werden. Der Stent 1 nach dem zweiten Ausführungsbeispiel unterscheidet sich von dem Stent nach dem ersten Ausführungsbeispiel lediglich dadurch, dass der Fixierabschnitt 3 nicht konisch zunimmt, sondern etwa parabolisch verjüngt ausgebildet ist, wobei er sich mit zunehmender Nähe zum Crimpkörper 5 stärker verjüngt.

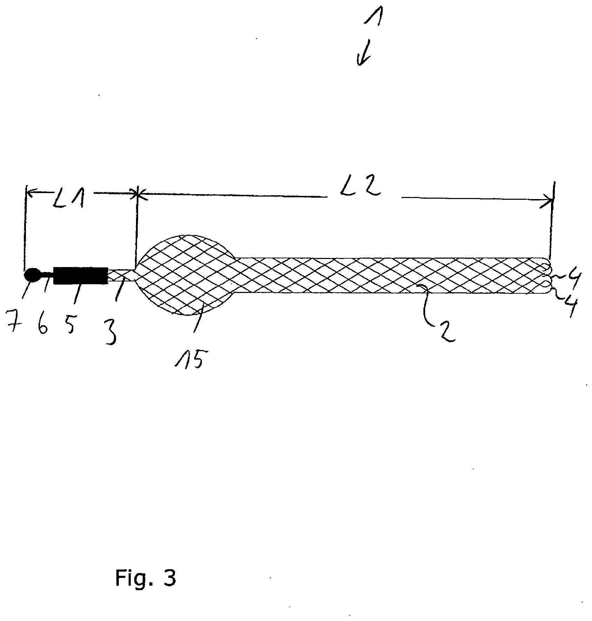

Fig. 3 zeigt ein erfindungsgemäßes Ausführungsbeispiel des Stents 1, wobei wiederum gleiche Teile wie in dem vorbeschriebenen Ausführungsbeispiel mit gleichen Bezugszeichen bezeichnet sind. Dieser Stent 1 unterscheidet sich von den obigen Ausführungsbeispielen dadurch, dass der proximale Endbereich des Stützkörpers 2 einen aufgeweiteten Abschnitt 15 aufweist. Der aufgeweitete Abschnitt 15 ist im vorliegenden Ausführungsbeispiel etwa kugelförmig aufgeweitet. Die Form des kugelförmig aufgeweiteten Abschnitts kann etwas von einer exakten Kugelform abweichen, z.B. oval ausgeführt sein. Wesentlich ist, dass der aufgeweitete Abschnitt keine Kanten bildet, die gegen die Nasenflügel drücken, sondern durch seine abgerundete Form von innen sanft den jeweiligen Nasenflügel abstützt. Dieser kugelförmig aufgeweitete Abschnitt sollte einen Durchmesser von zumindest 10 mm, vorzugsweise 12 mm und insbesondere 13 mm aufweisen, um eine wirksame Aufweitung der Nasenflügel zu bewirken. Der Durchmesser sollte jedoch nicht zu groß sein, damit kein starker Druck auf die Nasenflügel entsteht. Ein maximaler Durchmesser von 20 mm, insbesondere von 17 mm bzw. 15 mm ist daher zweckmäßig. Der übrige Stützkörper 2 ist daher um diesen Bereich kürzer ausgebildet im Vergleich zu den oben erläuterten Ausführungsbeispielen, so dass die gesamte Länge in etwa gleich bleibt.

Dieser kugelförmig aufgeweitete Abschnitt 15 ist vorgesehen, um im Bereich unmittelbar hinter der Nasenöffnung angeordnet zu werden, damit die Nasenflügel gegen einen Kollaps abgestützt werden.

Insbesondere bei der Ausführungsform mit aufgeweitetetem Abschnittt kann ein Verzicht auf einen Fixierabschnitt vorteilhaft sein, weil durch den kugelförmig aufgeweiteten Abschnitt 15 bereits eine ausreichende Fixierung in der Nase erfolgen kann, so dass ein Haltelement nicht unbedingt notwendig ist.

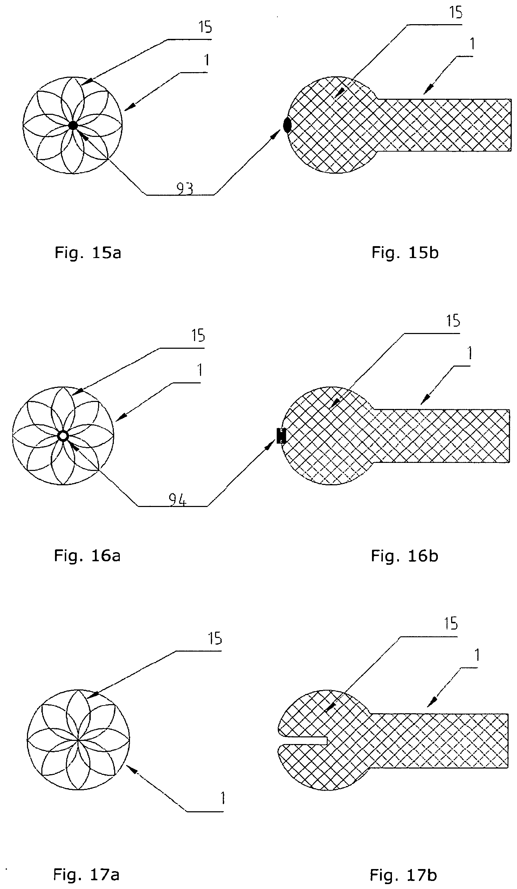

Nachfolgend werden einige Ausführungsformen des Stents ohne Fixierabschnitt bzw. ohne Kupplungselement anhand der Figuren 15, 15b bis 17a, 17b erläutert.

Bei der Ausführungsform gemäß Fig. 15a, 15b ist der aufgeweitete Bereich 15 derart ausgebildet, dass die Enden der Drähte in einem Kunststofftropfen 93 zusammengeführt sind. Der Kunststofftropfen wird vorzugsweise als flüssiges Polymer oder flüssiger Klebstoff auf die freien Enden der Drähte aufgetragen und so lange in Form gehalten, bis der Kunststoff ausgehärtet ist. Nach der Härtung sitzen die Enden der Drähte fest und es kann weder das Geflecht noch das damit in Berührung kommende Körpergewebe von scharfkantigen Spitzen beschädigt werden.

Bei einer weiteren Ausführungsform (Fig. 16a, 16b) werden die Enden der Drähte durch einen nietenähnlichen Ring 94 (ein oder zweiteilig) festgehalten. Hierfür werden die Enden der Drähte in einen umlaufenden Spalt am nietenähnlichen Ring hineingeführt und anschließend wird dieser zusammengedrückt bzw. gecrimpt.

Gemäß einer weiteren Ausführungsform (Fig. 17a, 17b) sind die Drähte durch Flechten und Wärmebehandlung zusammengefasst, wobei die Enden der Drähte in den Innenbereich des kugelförmig aufgeweiteten Bereichs zurückgeführt sind. Hierfür kann das proximale Ende des Geflechts mit Zwirbeln ausgestattet werden, sodass Verzerrungen und Ausfransungen ausgeschlossen werden können. Zusätzlich können die Enden der Drähte z.B. mit einer Hülse gecrimpt und damit befestigt werden.

Bei einem Stent ohne Kupplungselement wird der Einführschlauch über eine Einführstange, die keine rohrförmige Buchse enthält, oder über ein Einführrohr, das keine Durchgangsöffnungen und keinen Schlitz enthält, hinweg abgezogen, wobei sich der Stent im Nasengang entfaltet und positioniert.

In Fig. 3 ist das Drahtgeflecht nur schematisch dargestellt. Eine solche Form kann beispielsweise durch Flechten eines zylinderförmigen Stents aus einem Nitinoldraht hergestellt werden, wobei der zylinderförmige Stent auf eine Form aufgezogen wird, die der Form des Stützkörpers 2 entspricht und einen kugelförmigen Abschnitt und einen zylinderförmigen Abschnitt aufweist. Durch eine spezielle Temperaturbehandlung, die in der Fachwelt als Glühen bekannt ist, wird dann die Form des Formkörpers in dem Geflecht aus Nitinoldraht eingeprägt. Nach Herausnehmen des Formkörpers verbleibt der Stent in dieser Form.

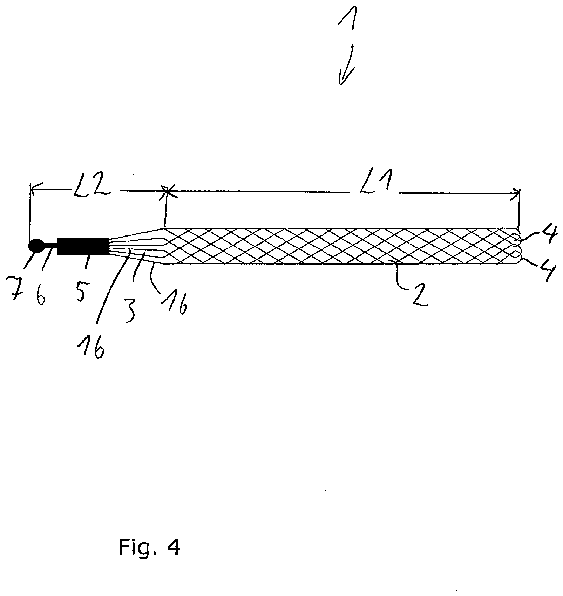

Fig. 4 zeigt ein weiteres Ausführungsbeispiel eines nicht-erfindungsgemäßen Stents 1, wobei wiederum gleiche Teile mit gleichen Bezugszeichen bezeichnet sind und nicht nochmals erläutert werden. Dieser Stent unterscheidet sich von den drei oben beschriebenen Ausführungsbeispielen dadurch, dass der Fixierabschnitt 3 nicht geflochten ist, sondern die Drähte verlaufen entweder einzeln in etwa parallel vom Stützkörper 2 zum stiftförmigen Körper bzw. Crimpkörper 5 oder die Drähte sind verzwirbelt und die verzwirbelten Stränge 16 laufen etwa parallel vom Stützkörper 2 zum Crimpkörper 5. Hierbei können jeweils zwei, drei oder vier Drähte zu einem verzwirbelten Strang 16 verzwirbelt sein. Der Vorteil dieses Fixierabschnittes 3 liegt darin, dass er eine hohe Flexibilität besitzt und der Stent im Bereich des Fixierabschnittes 3 sehr biegsam ist. Dieser Fixierabschnitt 3 kann selbstverständlich auch mit dem aufgeweiteten Abschnitt 15 gemäß dem dritten Ausführungsbeispiel kombiniert werden.

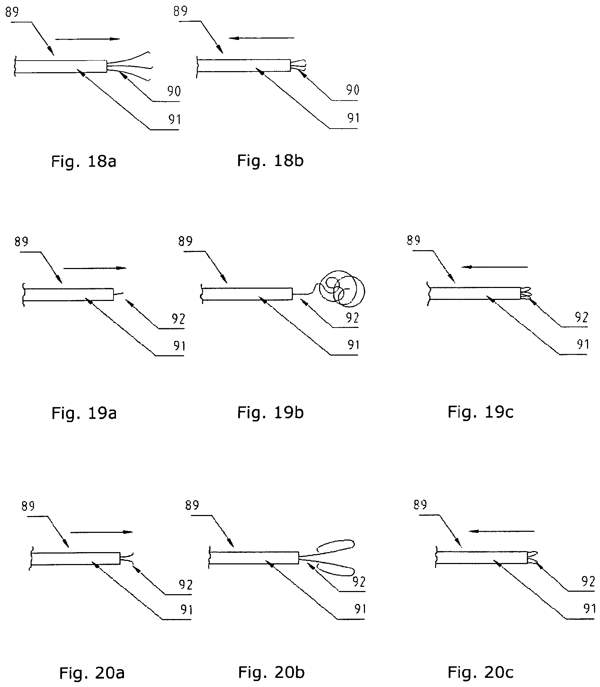

Ist der Stent ohne Kupplungselement, wie z.B. einer Kugelkupplung, ausgebildet, dann ist es zweckmäßig, ein Entfernungswerkzeug einzusetzen, um den Stent 1 wieder aus der Nase herauszuziehen. Die einfachste Ausführungsform ist ein dünner gestreckter Körper aus Kunststoff oder Metall, der am distalen Ende eine hakenförmige Krümmung aufweist (nicht dargestellt). Diese Krümmung wird in das Drahtgeflecht des Stents 1 eingeführt und der Stent mittels des Entfernungswerkzeugs aus der Nase herausgezogen
Alternativ können verschiedene andere Ausführungsformen von Entfernungswerkzeugen 89 eingesetzt werden, wie sie im Folgenden anhand der Figuren 18a bis 20c erläutert werden.

Eine zweite Ausführungsform eines Entfernungswerkzeug 89 (Fig. 18a, 18b) weist ähnlich einem Spannfutter eines Druckbleistiftes ausgebildete elastische Fangarme 90 auf, die verschieblich in einer Hülse 91 gelagert sind. Beim Ausschieben der Fangarme 90 aus der Hülse 91 gehen deren freien Enden aufgrund ihrer Federwirkung auseinander. Die Fangarme 90 sind an ihren freien Enden mit nach innen weisenden hakenförmigen Vorsprüngen ausgebildet. Mit diesen hakenförmigen Vorsprüngen werden sie in das Geflecht des Stents 1 eingeführt. Wenn die Fangarme im Geflecht liegen, werden die Fangarme 90 soweit in die Hülse 91 eingezogen, bis der Stent 1 fest gefasst ist. Dann wird der Stent aus der Nase herausgezogen.

In den Fig. 19a bis 19c ist eine dritte Ausführungsform eines Entfernungswerkzeuges 89 dargestellt, das wiederum eine Hülse 91 aufweist. In der Hülse 91 befindet sich verschieblich ein Fangdraht 92. Der Fangdraht 92 ist in der Hülse gestreckt, da der Innendurchmesser der Hülse 91 nur geringfügig größer als der Durchmesser des Fangdrahtes 92 ist. Der Fangdraht 92 ist derart vorgeformt, dass er sich beim Ausschieben aus der Hülse 91 aufwickelt und ein Knäuel bildet. Zum Fixieren an der Hülse wird das Entfernungswerkzeug 89 mit eingezogenem Fangdraht 92 an das proximale Ende des Stents positioniert. Anschließend wird der Fangdraht 92 aus der Hülse 91 geschoben. Der Fangdraht 92 bildet innerhalb des Geflechts des Stents 1 das Knäuel, womit auf den Stent eine zum Herausziehen aus der Nase ausreichende Zugkraft ausgeübt werden kann. Dadurch wird der Stent 1 aus der Nase entfernt.

Eine vierte Ausführungsform eines Entfernungswerkzeug 89 (Fig. 20a bis 20c) ist ähnlich ausgebildet wie die in den Figuren 19a bis 19c gezeigte dritte Ausführungsform des Entfernungswerkzeugs 89. Die vierte Ausführungsform des Entfernungswerkzeuges 89 umfasst wiederum eine Hülse 91, wobei darin mehrere Fangdrähte 92 angeordnet sind, die jeweils schlaufenförmig vorgeformt sind. Zum Greifen des Stents wird das Entfernungswerkzeug 89 mit der Mündung der Hülse 91 am proximalen Ende des Stents positioniert. Anschließend werden die Fangdrähte 92 aus der Hülse 91 geschoben. Die Fangdrähte entfalten sich innerhalb des Geflechts des Stents 1,so dass der Stent aus der Nase heraus gezogen werden kann.

### Nachfolgend werden noch einige Halteelemente erläutert:

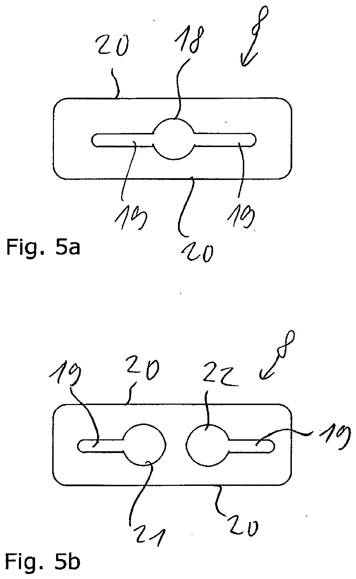

Figur 5a zeigt ein erstes Ausführungsbeispiel eines Halteelementes 8, das in der Draufsicht aus einem etwa rechteckförmigen Kunststoffplättchen ausgebildet ist. Die Ecken des Plättchens sind abgerundet. Das Plättchen weist eine Länge von etwa 3-4 cm und eine Breite von etwa 0,7-1,5 cm auf. Im Zentrum dieses plättchenförmigen Halteelements 8 ist eine kreisförmige Öffnung 18 ausgebildet, deren Durchmesser etwas größer als der Durchmesser der Kugelkupplung 7 des Stents 1 ist. Von der Öffnung 18 erstrecken sich zwei Schlitze 19, die parallel zur Längskante 20 des plättchenförmigen Halteelements 8 verlaufen. Die Schlitze 19 sind etwa in der Mitte der sich gegenüberliegenden Längskanten 20 angeordnet. Die Schlitze weisen eine Breite auf, die kleiner als der Durchmesser der Kugelkupplung 7 ist. Vorzugsweise sind die Schlitze als Einschnitte ausgebildet ohne Abstand zwischen den gegenüberliegenden Kanten des Einschnitts.

Eine Kugelkupplung 7 eines Stents 1 kann durch die Öffnung 18 des Halteelements 8 hindurchgeführt werden und der Stent kann in einen der beiden Schlitze 19 verschoben werden, wobei der Stent 1 sich mit seinem Stift 6 oder mit dem Drahtgeflecht durch den jeweiligen Schlitz 19 hindurch erstreckt. Ein zweiter Stent kann mit seiner Kugelkupplung 7 in die Öffnung 18 eingeführt und in den anderen Schlitz 19 verschoben werden. Die Stents 1 werden in das Halteelement 8 eingehängt, wenn sie bereits in den Nasengang eingeführt und der Einführschlauch sowie die Einführhilfe entfernt sind. Durch die Anordnung der Stents 1 in der Nase sind sie voneinander beabstandet, so dass zuverlässig verhindert wird, dass die Kugelkupplungen 7 in Richtung zur Öffnung 18 rutschen. Somit ist das Halteelement mit beiden Stents 1 verliersicher verbunden. Das Halteelement 8 bildet somit zwischen den beiden Stents 1 einen Bügel, der verhindert, dass die Stents zu tief in den Nasengang und insbesondere in den Rachen eindringen können.

Es ist vor allem auch zweckmäßig die Öffnung 18 mit einem am Halteelement 8 integrierten Trichter (nicht dargestellt) zu versehen, der das Einführen der Kugelkupplung 7 erleichtert. Beim Einführen der Kugelkupplung 7 in die Öffnung 18 ist der Stent 1 mit seinem Stützkörper im Nasengang angeordnet. Die Kugelkupplung 7 befindet sich somit unmittelbar vor der Nase und am Rande des Sichtfeldes des Benutzers. Daher ist ein derartiger Trichter sehr vorteilhaft, denn damit ist es möglich, das Halteelement an den Stent 1 zu koppeln, ohne dass man das Halteelement und den Fixierabschnitt 3 des Stents 1 sieht.

Figur 5b zeigt ein zweites Ausführungsbeispiel eines Halteelementes, das ähnlich wie das erste Ausführungsbeispiel aus Figur 5a ausgebildet ist, weshalb gleiche Teile mit gleichen Bezugszeichen bezeichnet werden und nicht nochmals erläutert werden. Dieses Halteelement ist wiederum aus einem rechteckförmigen Plättchen mit zwei Längskanten 20 ausgebildet. Es weist wiederum zwei Schlitze 19 auf. Die beiden Schlitze 19 münden jeweils in eine separate Öffnung 21, 22, die vom Zentrum des Plättchens etwas versetzt sind.

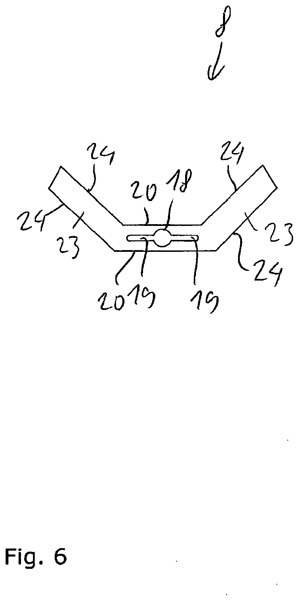

Ein drittes Ausführungsbeispiel des Halteelementes 8 ist in Figur 6 gezeigt. Dieses Halteelement ist ähnlich ausgebildet wie das in Figur 5a gezeigte Halteelement, weshalb gleiche Teile mit gleichen Bezugszeichen bezeichnet sind und nicht nochmals erläutert werden. An den zu den Längskanten senkrecht verlaufenden Stirnseiten des rechteckförmigen Plättchens ist jeweils ein Stützflügel 23 angeformt. Der Stützflügel ist in der Draufsicht etwa ein rechteckförmiges Plättchen, das sich mit seinen Längskanten 24 schräg bezüglich der Längskanten 20 des Hauptkörpers des Halteelements 8 erstreckt. Die beiden Stützflügel 23 sind bezüglich der Schlitze 19 jeweils zur gleichen Seite abgewinkelt. Die Stützflügel können nach dem Fixieren der Stents 1 einem Halteelement 8 unter die Nasenflügel geklemmt werden, so dass sie die Nasenflügel offen halten. Dieses Halteelement ist vor allem mit den Stents gemäß den Ausführungsbeispielen nach Figur 1, Figur 2 und Figur 3 kombinierbar, die keinen aufgeweiteten Abschnitt zum Offenhalten der Nasenflügel aufweisen.

Die Längskanten 24 der Stützflügel 23 begrenzen mit den Längskanten 20 des Hauptkörpers des Halteelements 8 einen Winkel im Bereich von 120° bis 150°.

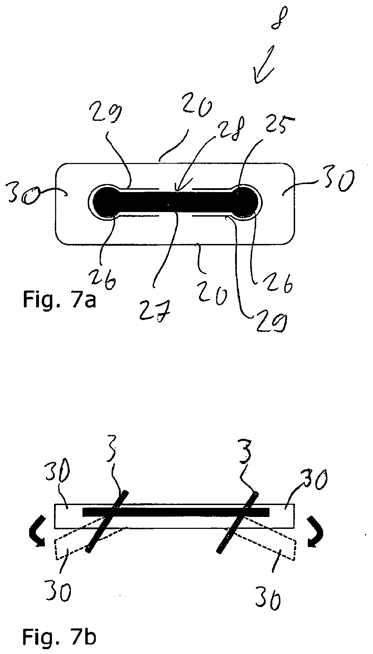

Figur 7a und 7b zeigen ein weiteres Ausführungsbeispiel eines Halteelements 8. Dieses Halteelement ist wiederum plättchenförmig und in der Draufsicht rechteckförmig mit zwei Längskanten 20 ausgebildet. Dieses Plättchen weist eine steife Einlage 25 auf, die in der Draufsicht etwa knochenförmig mit zwei kreisförmigen Abschnitten 26 und einem Verbindungssteg 27 zwischen den kreisförmigen Abschnitten 26 ausgebildet ist. Diese steife Einlage besteht beispielsweise aus Federstahl. Der übrige Bereich des plättchenförmigen Halteelements 8 ist aus einem flexiblen Kunststoffmaterial, insbesondere einem Elastomer, ausgebildet. Im Bereich der Längsmitte der steifen Einlage, der in Figur 7a mit dem Bezugszeichen 28 gekennzeichnet ist, ist die steife Einlage 25 mit dem übrigen Kunststoffmaterial integral verbunden, weshalb dieser Bereich als Verbindungsbereich 28 bezeichnet wird. Seitlich vom Verbindungsbereich 28 ist die steife Einlage 25 vollständig von dem flexiblen Kunststoffmaterial ausgestanzt, wie es in Figur 7a anhand zweier Stanzlinien 29 dargestellt ist. Das flexible Kunststoffmaterial bildet somit zwei flexible Zungen 30, die mit der steifen Einlage 25 im Verbindungsbereich 28 verbunden sind. Die flexiblen Zungen 30 weisen somit Öffnungen auf, deren Form komplementär zu den Endbereichen der steifen Einlage 25 ist.

Wie es in Figur 7b schematisch dargestellt ist, können die flexiblen Zungen 30 vom Endbereich der steifen Einlage 25 angehoben werden, so dass die in den Zungen 30 ausgebildeten Öffnungen freigelegt werden. Durch diese Öffnungen kann jeweils ein Fixierabschnitt 3 eines Stents 1 hindurchgeführt werden. Dieser Fixierabschnitt 3 wird dann zwischen den Endabschnitten der steifen Einlage 25 und der flexiblen Zunge 30 geklemmt und so mit dem Halteelement 8 verliersicher verbunden.

Dieses Halteelement 8 gemäß Figur 7a und 7b ist sehr einfach ausgebildet und kostengünstig herstellbar und bietet trotzdem eine sichere Klemmung der Fixierabschnitte 3 zweier Stents 1. Im Rahmen der Erfindung ist es selbstverständlich auch möglich andere Klemmelemente, insbesondere Klammern zu verwenden, die ein Federelement aufweisen, das zwei Klemmbacken beaufschlagt. Weiterhin kann das Halteelement 8 mit einer oder zwei Buchsen versehen sein, die so ausgebildet sind, wie die Buchse 10 der Einführstange 9. Auch mit diesen Buchsen ist eine Kopplung des bzw. der Stents an das Halteelement möglich.

Die Halteelemente gemäß Fig. 5b, Fig. 6 und Fig. 7a und 7b können auch im Bereich ihrer Öffnungen 18, 21, 22, 29 mit einem integrierten Trichter versehen sein.

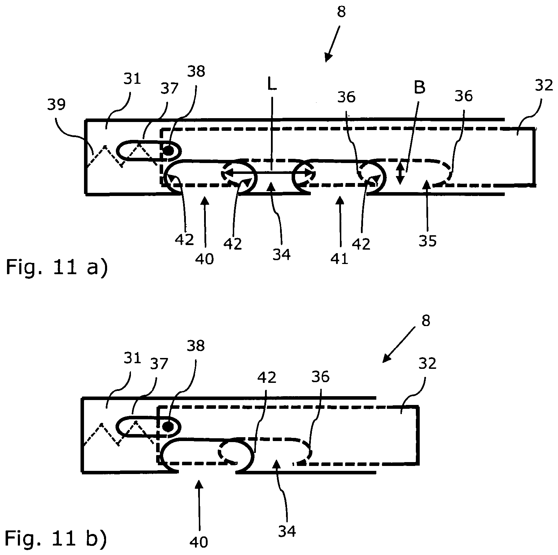

Fig. 11a zeigt ein weiteres Halteelement 8, das aus einer rohrförmigen, langgestreckten hohlen Hülse oder Gehäuse 31 und einem verschieblich im Gehäuse 31 gelagerten Einsatz 32 ausgebildet ist. Das Gehäuse 31 weist ein mit einer Stirnwandung 33 verschlossenes Ende und ein offenes Ende auf. Der Einsatz 32 ist an die Form des Gehäuses 31 angepasst, wobei das Gehäuse 31 und der Einsatz 32 vorzugsweise einen rechteckförmigen, insbesondere quadratischen, oder kreisförmigen Querschnitt aufweisen. Der Einsatz 32 kann ein massiver Kunststoff-Körper sein, der an einer Längsseite eine erste Ausnehmung 34 und eine zweite Ausnehmung 35 aufweist. Die Ausnehmungen 34, 35 erstrecken sich von der Längsseite mit einer vorbestimmten Tiefe B in den Einsatz 32 hinein. In Längsrichtung erstrecken sich die Ausnehmungen 34, 35 mit einer vorbestimmten Länge L, die vorzugsweise wesentlich größer als die Tiefe B ist. An den in Längsrichtung liegenden Enden der Ausnehmungen 34, 35 sind diese jeweils mit einem konkaven Bogen 36 geformt.

Das Gehäuse 31 weist zwei diametral gegenüberliegende Längsschlitze 37 auf, in welchen jeweils ein Ende eines mit dem Einsatz 32 verbundener Querstift 38 gelagert ist. Durch den Querstift 38 ist die Bewegung des Einsatzes 32 im Gehäuse 31 begrenzt, wobei bei einer maximal eingedrückten Stellung des Einsatzes 32 der Querstift 38 am zur Stirnwandung 33 weisenden Ende des Längsschlitzes 37 anschlägt und bei einer maximal ausgefahrenen Stellung des Einsatzes 32 der Querstift 38 an dem von der Stirnwandung 33 entfernten Ende des Längsschlitzes 37 anschlägt. Die Länge des Längsschlitzes 37 entspricht somit dem maximalen Weg des Einsatzes 32 im Gehäuse 31. Zwischen der Stirnwandung 33 und dem Einsatz 32 befindet sich ein Federelement 39, das den Einsatz 32 in seine maximal ausgefahrene Stellung drückt (Figur 11A).

Das Gehäuse 31 weist auch eine erste Ausnehmung 40 und eine zweite Ausnehmung 41 auf, die etwa die gleiche Form wie die erste Ausnehmung 34 und die zweite Ausnehmung 35 des Einsatzes 32 aufweisen und in der maximal eingedrückten Stellung des Einsatzes 32 mit den Ausnehmungen 34, 35 fluchten. Die Ausnehmungen 40, 41 weisen somit auch konkave Bögen 42 auf, die die Ausnehmungen in ihrer Längsrichtung begrenzen.

In einer eingedrückten Stellung des Einsatzes 32, in der die ersten Ausnehmungen 34, 40 und die zweiten Ausnehmungen 35, 41 etwa fluchten, ergibt sich somit eine durchgehende Ausnehmung, in welche jeweils ein Fixierabschnitt 3 eines Stents 1 eingefügt werden kann. Wird dann der Einsatz 32 freigegeben, so wird er durch das Federelement 39 von der Stirnwandung 33 weggedrückt, wodurch die konkaven Bögen 36 der Ausnehmungen des Einsatzes und die konkaven Bögen 42 des Gehäuses 31 aufeinander zu bewegt werden und zwischen sich die Fixierabschnitte 3 der Stents 1 einklemmen.

Der Einsatz 32 wird an seinem am offenen Ende des Gehäuses 31 vorstehenden Abschnitt betätigt, um gegen das Federelement 39 gedrückt zu werden.

Figur 11b zeigt ein weiteres Haltelement 8, das ähnlich wie das Halteelement 8 gemäß Fig. 11a ausgebildet ist, jedoch lediglich eine einzige Ausnehmung 34 im Einsatz 32 und eine einzige Ausnehmung 40 im Gehäuse 31 aufweist, die bei eingedrücktem Einsatz 32 zueinander fluchten. Mit diesem Halteelement kann ein einzelner Stent fixiert werden. Zwei solche Halteelemente können mit einem biegsamen Band verbunden sein, so dass mit einem jedem der Halteelemente 8 ein Stent 1 fixiert werden kann. Die Verwendung zweier Haltelemente 8, die mit einem biegsamen Band miteinander verbunden sind, hat den Vorteil, dass zunächst ein Halteelement der beiden Halteelemente an einem der beiden Stents gekoppelt wird und dann das andere Halteelement unabhängig vom ersten Halteelement bezüglich des zweiten Stents ausgerichtet und mit diesem gekoppelt werden kann.

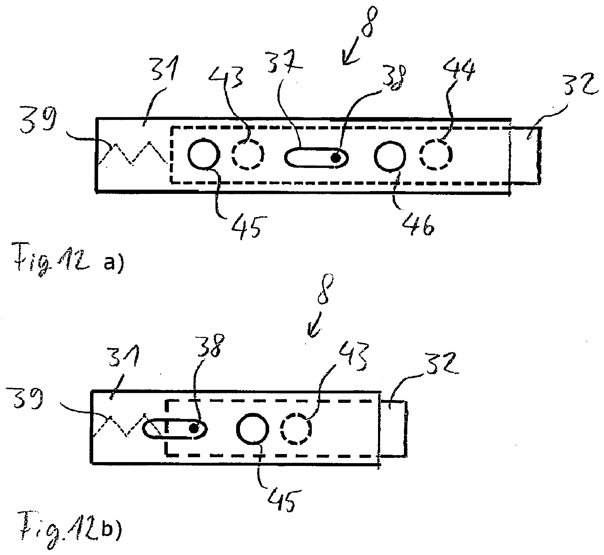

Figur 12a zeigt ein weiteres Halteelement 8, das ähnlich wie das Halteelement aus Fig. 11a ausgebildet ist, weshalb gleiche Teile mit den gleichen Bezugszeichen bezeichnet sind und nicht nochmals erläutert werden. Das Halteelement gemäß Fig. 12a unterscheidet sich von demjenigen gemäß Fig. 11a alleine durch das Vorsehen von Durchgangsöffnungen 43, 44 im Einsatz 32 und Durchgangsöffnungen 45, 46 im Gehäuse 31, die bei eingedrücktem Einsatz zueinander fluchten. Im vorliegenden Ausführungsbeispiel sind die Durchgangsöffnungen 43 bis 46 kreisförmig ausgebildet. Im Rahmen der Erfindung ist es selbstverständlich möglich, anstelle von kreisförmigen Öffnungen Langlöcher vorzusehen, die in Längsrichtung des Gehäuses 31 bzw. in Längsrichtung des Einsatzes 32 ausgerichtet sind.

In Fig. 12b ist ein Halteelement 8 gezeigt, das ähnlich zu dem Halteelement gemäß Fig. 12a ausgebildet ist, wobei dieses Halteelement im Einsatz 32 und im Gehäuse 31 jeweils eine Durchgangsöffnung 43, 45 aufweist, die bei eingedrücktem Einsatz 32 zueinander fluchten. Das Halteelement 8 gemäß Fig. 11b dient zum Fixieren eines einzelnen Stents. Zwei dieser Halteelemente können mit einem biegsamen Band verbunden sein, um zwei Stents zu fixieren.

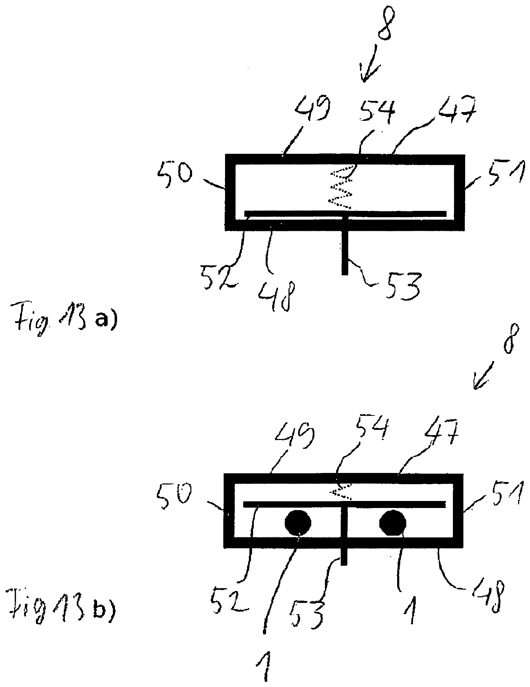

Figur 13a zeigt schematisch grob vereinfacht ein weiteres Halteelement 8, das einen rechteckförmigen Rahmen 47 mit zwei Längsstreben 48, 49 und zwei Querstreben 50, 51 aufweist. In diesem Rahmen 47 ist eine Stempelplatte 52 angeordnet, die mittig mit einer Führungsstange 53 verbunden ist, die senkrecht auf der Stempelplatte 52 steht. Auf der von der Führungsstange 53 abgewandten Seite ist ein Federelement 54 zwischen der Stempelplatte und der Längsstrebe 49 angeordnet. Dieses Federelement drückt die Stempelplatte gegen die Längsstrebe 48. Die Führungsstange 53 erstreckt sich durch eine entsprechende Bohrung in der Längsstrebe 48, so dass die Stempelplatte 52 parallel zu den Längsstreben 48, 49 geführt ist.

Zum Verbinden dieses Haltelementes 8 mit den Fixierabschnitten 3 zweiter Stents 1 wird die Führungsstange gegen die Wirkung des Federelementes 54 in den Rahmen 47 eingedrückt, so dass die Stempelplatte 52 von der Längsstrebe 48 angehoben wird. In dem Bereich zwischen der Stempelplatte 52 und der Längsstrebe 48 können dann die beiden Fixierabschnitte 3 der Stents 1 eingeführt werden. Durch Freigeben der Führungsstange 53 wird die Stempelplatte 52 gegen die Längsstrebe 48 gedrückt, wodurch die Fixierabschnitte 3 des Stents 1 zwischen den Stempelplatten 52 und den Längsstreben 48 eingeklemmt werden.

Der Vorteil dieses Halteelementes 8 liegt darin, dass der geöffnete Bereich zwischen der Stempelplatte 52 und den Längsstreben 48 sehr groß ist, so dass es einfach ist, die Fixierabschnitte 3 einzuführen.

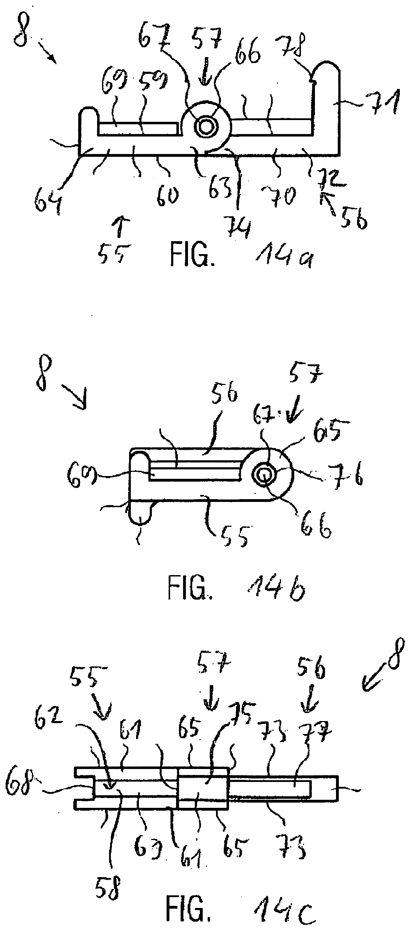

In der PCT/EP 2010/004687 ist eine Fixiervorrichtung beschrieben, die hier gleichermaßen als Halteelement 8 verwendet werden kann (Figur 14a bis 14c). Diesbezüglich wird auf dieses Dokument verwiesen. Dieses Halteelement 8 umfasst eine erste und eine zweite Klemmbacke 55, 56, die über ein Gelenk 57 miteinander verbunden sind.

Die erste Klemmbacke 55 wird im Folgenden als Basisbacke bezeichnet. Die Basisbacke 55 weist eine Basiswandung 58 auf. Die Basiswandung umfasst eine Oberseite 59, eine Unterseite 60 und von der Basiswandung 58 erstrecken sich an deren Längsseiten zwei Seitenwandungen 61 nach oben, so dass die Basiswandung 58 und die zwei Seitenwandungen 61 eine U-förmige Ausnehmung 62 begrenzen. Die beiden gegenüberliegenden Enden der quaderförmigen Basiswandung 58 werden als Gelenkseite 63 und Verschlussseite 64 bezeichnet.

An der Gelenkseite 63 der Basiswandung 58 sind zwei Ringscheibenelemente 65 beabstandet voneinander angeformt, die bündig zu den Außenflächen der Seitenwandungen 61 ausgebildet sind. Die Ringscheibenelemente 65 weisen jeweils eine kreisförmige Durchgangsöffnung 66 auf, die zueinander fluchten. Die Durchgangsöffnungen 66 sind jeweils zur Aufnahme einer Rohrwelle 67 ausgebildet. Die Rohrwelle 67 ist drehbar in den Durchgangsöffnungen 66 angeordnet und bündig zu den Außenflächen der Seitenwandungen 61 ausgebildet. Über die Rohrwelle 67 ist die erste Klemmbacke 55 gelenkig mit der zweiten Klemmbacke 56 verbunden.

An der Verschlussseite 64 erstrecken sich die Seitenwandungen 61 über den stirnseitigen Rand der Basiswandung 58 hinaus, so dass sie in der Draufsicht auf die Basisbacke 55 die U-förmige Ausnehmung begrenzen. Die Seitenwandungen sind in diesem Bereich ein Stück nach oben gezogen.

Die stirnseitige Kante der Basiswandung 58 an der Unterseite 60 wird im Folgenden als Fixierkante 68 bezeichnet.

In der U-förmigen Ausnehmung bzw. Nut 62 ist ein quaderförmiger Fixierblock 69 angeordnet. Der quaderförmige Fixierblock 69 ragt in etwa 3 mm auf der Oberseite 59 der Basiswandung 58 heraus. Der quaderförmige Fixierblock 69 ist aus einem Kunststoff, insbesondere Silikon, ausgebildet und bildet ein Fixierelement aus.

Die zweite Klemmbacke 56 ist L-förmig ausgebildet und weist einen quaderförmigen Querschnitt auf. Die zweite Klemmbacke 56 umfasst einen langen Schenkel 70 und einen daran im rechten Winkel angeformten kurzen Schenkel, der im Folgenden als Fixierschenkel 71 bezeichnet wird. Der lange Schenkel 70 ist wiederum nutförmig mit einer Basiswandung 72 und zwei Seitenwandungen 73 ausgebildet.

Das dem Fixierschenkel 71 gegenüberliegende Ende des langen Schenkels 70 wird als Gelenkseite 74 bezeichnet. An diese Gelenkseite 74 ist ein rohrförmiger Gelenkkörper 75 angeformt. Der Gelenkkörper 75 bildet ein Rohr mit zwei Stirnseiten und einem kreisförmigen Durchgang, wobei die Länge des Rohrs der lichten Weite zwischen den beiden Ringscheibenelementen 65 entspricht. In der Durchgangsöffnung des Gelenkkörpers ist die Rohrwelle 67 angeordnet. Die Rohrwelle 67 lagert drehbar in den Durchgangsöffnungen 66 der Ringscheibenelemente 65 und in der Durchgangsöffnung des Gelenkkörpers 75.

Im Fixierschenkel 71 ist ein Durchgangsloch ausgebildet (nicht dargestellt), das mit der Nut des langen Schenkels 70 fluchtet. Im rohrförmigen Gelenkkörper 75 ist eine Ausnehmung eingebracht, die auch mit der Nut des langen Schenkels 70 fluchtet. Im Durchgangsloch, der Nut des langen Schenkels 70 und der Ausnehmung des Gelenkkörpers 75 ist ein Fixierschlauch 77 aufgenommen. Der Fixierschlauch 77 bzw. das Fixierelement ist aus Kunststoff, insbesondere Silikon, ausgebildet.

Am freien Ende des Fixierschenkels 71 ist eine in Richtung zum Gelenkkörper 75 zeigende Rastnase 78 ausgebildet. Die Rastnase 78 ist derart ausgebildet, dass sie die Fixierkante 71 hintergreift, wenn die beiden Schenkel zusammengedrückt werden. Die Rastnase 78 rastet dann hinter der Fixierkante 68 ein und hält das Halteelement 8 in einem geschlossenen Zustand. Die Rastnase 78 und die Fixierkante bilden so ein Verschlusselement aus. Im geschlossenen Zustand des Halteelementes sind die Basisbacke 55 und die zweite Klemmbacke 56 etwa parallel zueinander angeordnet, wobei der Fixierblock 69 und der Fixierschlauch 77 aufeinandergepresst werden.

Zwischen dem Fixierblock 69 der Basisbacke 55 und dem Fixierschlauch 77 der zweiten Klemmbacke 56 kann ein Fixierabschnitt 3 eines Stents 1 angeordnet werden. Es ist auch möglich, mit diesem Halteelement gleichzeitig zwei Fixierabschnitte 3 zweier Stents gleichzeitig zwischen einem Fixierblock 69 und einem Fixierschlauch 77 zu fixieren. Dazu ist es zweckmäßig, dass sich der Fixierblock 69 und der Fixierschlauch 77 jeweils über eine Länge von zumindest 2 bis 3 cm erstrecken.

Im Rahmen der Erfindung kann es auch zweckmäßig sein, ein derartiges Halteelement in miniaturisierter Ausführungsform auszubilden, wobei sich die Klemmbacken 55, 56 beispielsweise lediglich über eine Länge von etwa 1 cm erstrecken. Zwei derartige Halteelemente sind dann mit einem biegsamen Band miteinander verbunden, so dass mit jeweils einem der Halteelemente ein Stent fixiert werden kann.

Die oben erläuterten Halteelemente 8 sind jeweils zum Sichern zweier Stents 1 ausgebildet. Im Rahmen der Erfindung ist es selbstverständlich auch möglich, ein Halteelement 8 zum Fixieren lediglich eines einzigen Stents auszubilden. Hierbei ist es zweckmäßig, wenn sich das Halteelement zumindest in eine Richtung soweit erstreckt, dass das Halteelement nicht durch ein Nasenloch hineingezogen werden kann.

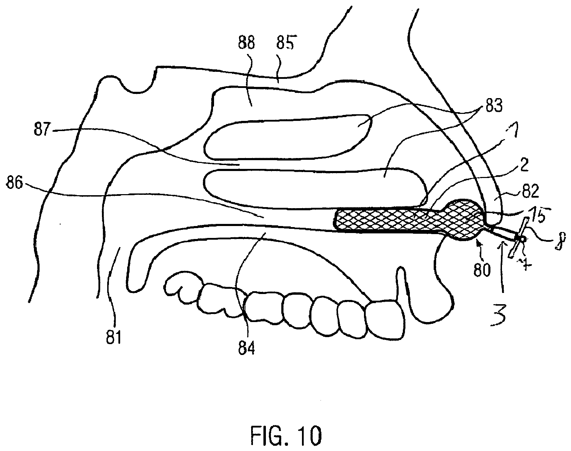

Fig. 10 zeigt in einer schematischen Darstellung den erfindungsgemäßen Stent 1 nach Figur 3 eingesetzt in eine Nasenhöhle 79. Die Nasenhöhle 79 erstreckt sich von einer Nasenöffnung 80 bzw. einem Nasenloch 80 in Richtung zu einem Nasenrachenraum 81. Im Bereich der Nase ist die Nasenhöhle durch Nasenflügel 82 begrenzt. In der Nasenhöhle befinden sich zwei Nasenmuscheln 83, die im Bereich zwischen dem Gaumen 84 und der Siebbeinplatte 85 der Nasenhöhle 79 angeordnet sind. Zwischen dem Gaumen 84 und der unteren Nasenmuschel 83 ist ein unterer Nasengang 86, zwischen den beiden Nasenmuscheln 83 ein mittlerer Nasengang 87 und zwischen der oberen Nasenmuschel 83 und der Siebbeinplatte 85 der Nasenhöhle ein oberer Nasengang 88 ausgebildet.

Sind die Nasenmuscheln angeschwollen, dann kann ein Nasengang mit dem erfindungsgemäßen Stent offengehalten werden. Der Stent 1 wird vor allem im unteren Nasengang 86 eingesetzt. Es ist jedoch auch möglich, ihn im mittleren Nasengang 87 bzw. oberen Nasengang 88 einzusetzen. Vor allem der untere Nasengang 86 weist in seinem hinteren Bereich eine besondere Empfindlichkeit auf. In diesem Bereich sollte der Stützkörper 2 des Stents 1 möglichst nicht enden, da die rückwärtige Kante unangenehm auf die Nasenmuschel 83 drücken könnte. Es ist deshalb zweckmäßig, den Stützkörper so anzuordnen, dass er nur den vorderen Bereich des Nasengangs schient oder sich vollständig durch den Nasengang hindurch erstreckt.

Es ist auch vorteilhaft, dass der erfindungsgemäße Stent biegsam ist, so dass er sich an die Konturen der Nasengänge anschmiegen kann. Dies wird einerseits durch die Elastizität des Drahtes und andererseits durch die Ausbildung als Geflecht bewerkstelligt.

Fig. 10 zeigt lediglich einen Stent 1, der mit einem Haltelement 8 fixiert ist.

Im Rahmen der Erfindung ist es möglich, lediglich einen einzigen Stent zu verwenden. In der Regel werden jedoch zwei Stents 1 eingesetzt. Die beiden Stents sind normalerweise gleich ausgebildet. In speziellen Anwendungen kann es jedoch auch sinnvoll sein, zwei Stents unterschiedlicher Größe (unterschiedlicher Länge und/oder mit unterschiedlichem Durchmesser und/oder unterschiedlicher Bauart) zu verwenden. So kann es auch manchmal sinnvoll sein, einen erfindungsgemäßen Stent zum Schienen eines Nasenganges mit einem Stent zum Schienen des Atemweges im Rachenraum, wie er z.B. aus der WO 2007/065408 A2 bekannt ist, zu kombinieren und gleichzeitig einzusetzen und mit einem Halteelement 8 zu fixieren.

**Bezugszeichenliste**

| | | | |
|---|---|---|---|
| 1 | Stent | 48 | Längsstrebe |
| 2 | Stützkörper | 49 | Längsstrebe |
| 3 | Fixierabschnitt | 50 | Querstrebe |
| 4 | rundes Ende | 51 | Querstrebe |
| 5 | Krimpkörper | 52 | Stempelplatte |
| 6 | Stift | 53 | Führungsstange |
| 7 | Kugelkupplung | 54 | Federelement |
| 8 | Halteelement | 55 | erste Klemmbacke |
| 9 | Einführstange | 56 | zweite Klemmbacke |
| 10 | Buchse | 57 | Gelenk |
| 11 | Durchgangsöffnung | 58 | Basiswandung |
| 12 | Schlitz | 59 | Oberseite |
| 13 | Einführschlauch | 60 | Unterseite |
| 14 | Einführspitze | 61 | Seitenwandung |
| 15 | aufgeweiteter Abschnitt | 62 | Ausnehmung |
| 16 | verzwirbelter Strang | 63 | Gelenkseite |
| 17 | Fase | 64 | Verschlussseite |
| 18 | Öffnung | 65 | Ringscheibenelement |
| 19 | Schlitz | 66 | Durchgangsöffnung |
| 20 | Längskante | 67 | Rohrwelle |
| 21 | Öffnung | 68 | Fixierkante |
| 22 | Öffnung | 69 | Fixierblock |
| 23 | Stützflügel | 70 | langer Schenkel |
| 24 | Längskante | 71 | Fixierschenkel |
| 25 | steife Einlage | 72 | Basiswandung |
| 26 | kreisförmiger Abschnitt | 73 | Seitenwandung |
| 27 | Verbindungssteg | 74 | Gelenkseite |
| 28 | Verbindungsbereich | 75 | Gelenkkörper |
| 29 | Stanzlinie | 76 | Durchführungsöffnung |
| 30 | flexible Zunge | 77 | Fixierschlauch |
| 31 | Gehäuse | 78 | Rastnase |
| 32 | Einsatz | 79 | Nasenhöhle |
| 33 | Stirnwandung | 80 | Nasenöffnung/Nasenloch |
| 34 | erste Ausnehmung | 81 | Nasenrachenraum |
| 35 | zweite Ausnehmung | 82 | Nasenflügel |
| 36 | konkaver Bogen | 83 | Nasenmuschel |
| 37 | Längsschlitz | 84 | Gaumen |
| 38 | Querstift | 85 | Siebbeinplatte |
| 39 | Federelement | 86 | unterer Nasengang |
| 40 | erste Ausnehmung | 87 | mittlerer Nasengang |
| 41 | zweite Ausnehmung | 88 | oberer Nasengang |
| 42 | konkaver Bogen | 89 | Entfernungswerkzeug |
| 43 | Durchgangsöffnung | 90 | Fangarm |
| 44 | Durchgangsöffnung | 91 | Hülse |
| 45 | Durchgangsöffnung | 92 | Fangdraht |
| 46 | Durchgangsöffnung | 93 | Kunststofftropfen |
| 47 | Rahmen | 94 | Ring |

## Patentansprüche

1. Stent zum Schienen eines Nasenganges, wobei der Stent (1) aus einem geflochtenen, rohrförmigen Stützkörper (2) ausgebildet ist,
**dadurch gekennzeichnet,**
**dass** der Stützkörper (2) im Wesentlichen zylinderförmig ausgebildet ist und an seinem proximalen Ende einen aufgeweiteten Abschnitt (15) aufweist, der etwa kugelförmig aufgeweitet ist, wobei der Stützkörper im unbelasteten Zustand einen Durchmesser von 4 bis 20 mm und eine Länge von 25 bis 100 mm aufweist.

2. Stent nach Anspruch 1,
**dadurch gekennzeichnet,**
**dass** der aufgeweitete Abschnitt (15) einen Durchmesser von zumindest 10 mm, insbesondere 10-20 mm, aufweist.

3. Stent nach Anspruch 1 oder 2,
**dadurch gekennzeichnet,**
**dass** der Stent (1) einen Fixierabschnitt (3) aufweist, der am proximalen Ende des Stents (1) angeordnet ist und zum Fixieren des Stents (1) in einem Nasengang eines Benutzers ausgebildet ist, wobei beim Fixieren der Fixierabschnitt (3) sich aus dem Nasenloch des Benutzers heraus erstreckt und außerhalb der Nase fixierbar ist.

4. Stent nach einem der Ansprüche 1 bis 3,
**dadurch gekennzeichnet,**
**dass** der Stent in einem Einführschlauch (14) komprimierbar und beim Freisetzen aus dem Einführschlauch selbstexpandierend ausgebildet ist.

5. Stent nach einem der Ansprüche 1 bis 4,
**dadurch gekennzeichnet,**
**dass** zumindest der Stützkörper (2) aus einem Drahtgeflecht ausgebildet ist,

6. Stent nach einem der Ansprüche 1 bis 5,
**dadurch gekennzeichnet,**
**dass** der Stützkörper (2) aus einem elastischen Draht mit einem Durchmesser von 0,001 mm bis 2 mm, insbesondere mit einem Durchmesser von 0,05 mm bis 0,5 mm, und vorzugsweise mit einem Durchmesser von 0,07 mm bis 0,2 mm ausgebildet ist, wobei der Draht insbesondere ein Metalldraht und vorzugsweise ein Nitinoldraht oder ein Stahldraht ist.

7. Stent nach einem der Ansprüche 1 bis 6,
**dadurch gekennzeichnet,**
**dass** der Stent (1) einen Stützkörper (2) aufweist, der im unbelasteten Zustand einen Durchmesser von maximal 15 mm und/oder eine Länge von entweder 30 bis 50 mm oder von 70 bis 100mm aufweist.

8. Stent nach einem der Ansprüche 1 bis 7,
**dadurch gekennzeichnet,**
**dass** das distale Ende des Stützkörpers (2) verjüngt ist.

9. Stent nach einem der Ansprüche 1 bis 8,
**dadurch gekennzeichnet,**
**dass** der Fixierabschnitt (3) ein Kupplungselement (7), insbesondere eine Kugelkupplung, zum Fixieren des Stents (1) an einem Halteelement (8) und/oder an einer Einführstange (9) aufweist.

10. Stent nach Anspruch 9,
**dadurch gekennzeichnet,**
**dass** ein Halteelement (8) vorgesehen ist, wobei das Haltelement (8) zumindest ein Gegenkupplungselement aufweist, das mit dem Kupplungselement (7) des Stents (1) koppelbar ist.

11. System zum Schienen eines Nasenganges, umfassend
einen Stent (1) nach einem der Ansprüche 1 bis 9 und eine Einführstange (9) und einen Einführschlauch (13).

12. System zum Schienen eines Nasengangs nach Anspruch 11,
**dadurch gekennzeichnet,**
**dass** der Stent lösbar mit der Einführstange (9) verbindbar ist und/oder das System ein Entfernungswerkzeug (89) zum Entfernen des Stents (1) aus einem Nasengang umfasst, wobei das Entfernungswerkzeug an den Stent (1) koppelbar ist.

## Claims

1. A stent for splinting a nasal passage, the stent (1) being designed of a braided, tubular support body (2),
**characterized in that**
the support body (2) is substantially cylindrical and has a widened area (15) at the proximal end thereof, said widened area being widened approximately spherically, wherein, in an unloaded state, the support body has a diameter of 4 to 20 mm and a length of 25 to 100 mm.

2. The stent according to claim 1,
**characterized in that**
the widened area (15) has a diameter of at least 10 mm, in particular 10-20 mm.

3. The stent according to claim 1 or 2,
**characterized in that**
the stent (1) has a fixing section (3), which is arranged at the proximal end of the stent (1) and is designed to fix the stent (1) in a nasal passage of a user, wherein during fixing the fixing section (3) protrudes from the nostril of the user and can be fixed outside the nose.

4. The stent according to any of claims 1 to 3,
**characterized in that**
the stent is compressible in an insertion tube (14) and is self-expanding when it is released from the insertion tube.

5. The stent according to any of claims 1 to 4,
**characterized in that**
at least the support body (2) is made from a wire braid.

6. The stent according to any of claims 1 to 5,
**characterized in that**
the support body (2) is made from an elastic wire with a diameter of 0.001 mm to 2 mm, in particular with a diameter of 0.05 mm to 0.5 mm and preferably with a diameter of 0.07 mm to 0.2 mm, the wire being in particular a metal wire and preferably a nitinol wire or a steel wire.

7. The stent according to any of claims 1 to 6,
**characterized in that**
the stent (1) has a support body (2), which, in an unloaded state, has a maximum diameter of 15 mm and/or a length of either 30 to 50 mm or of 70 to 100 mm.

8. The stent according to any of claims 1 to 7,
**characterized in that**
the distal end of the support body (2) is tapered.

9. The stent according to any of claims 1 to 8,
**characterized in that**
the fixing section (3) has a coupling element (7), in particular a ball-shaped coupling, for fixing the stent (1) at a holding element (8) and/or at an inserting rod (9).

10. The stent according to claim 9,
**characterized in that**
a holding element (8) is provided, the holding element (8) having at least one matching coupling element that can be coupled to the coupling element (7) of the stent (1).

11. A system for splinting a nasal passage, comprising
a stent (1) according to any of claims 1 to 9 and an inserting rod (9) and an insertion tube (13).

12. The system for splinting a nasal passage according to claim 11,
**characterized in that**
the stent can be detachably connected to the inserting rod (9) and/or the system comprises a removal tool (89) for removal of the stent (1) from a nasal passage, wherein the removal tool can be coupled to the stent (1).

## Revendications

1. Stent destiné à servir d'attelle d'une voie nasale, dans lequel le stent (1) est réalisé à partir d'un corps de support tubulaire tressé (2),
**caractérisé par le fait**
**que** le corps de support (2) est réalisé sensiblement cylindrique et présente, à son extrémité proximale, un segment évasé (15) qui est évasé de manière environ sphérique, le corps de support présentant, à l'état non chargé, un diamètre de 4 à 20 mm et une longueur de 25 à 100 mm.

2. Stent selon la revendication 1,
**caractérisé par le fait**
**que** la partie évasée (15) présente un diamètre d'au moins 10 mm, en particulier de 10 à 20 mm.

3. Stent selon la revendication 1 ou 2,
**caractérisé par le fait**
**que** le stent (1) présente un segment de fixation (3) qui est disposé à l'extrémité proximale du stent (1) et est réalisé pour fixer le stent (1) dans une voie nasale d'un utilisateur, où, lors de la fixation du segment de fixation (3), le segment de fixation (3) s'étend en-dehors de la narine de l'utilisateur et peut être fixé à l'extérieur du nez.

4. Stent selon l'une des revendications 1 à 3,
**caractérisé par le fait**
**que** le stent peut être comprimé dans un flexible d'insertion (14) et est réalisé de manière auto-expansible lors de la libération du flexible d'insertion.

5. Stent selon l'une des revendications 1 à 4,
**caractérisé par le fait**
**qu'**au moins le corps de support (2) est réalisé en un treillis.

6. Stent selon l'une des revendications 1 à 5,
**caractérisé par le fait**
**que** le corps de support (2) est réalisé en un fil élastique d'un diamètre de 0,001 mm à 2 mm, en particulier d'un diamètre de 0,05 mm à 0,5 mm et de préférence d'un diamètre de 0,07 mm à 0,2 mm, où le fil est en particulier un fil métallique et de préférence un fil de nitinol ou un fil d'acier.

7. Stent selon l'une des revendications 1 à 6,
**caractérisé par le fait**
**que** le stent (1) présente un corps de support (2) qui présente, à l'état non chargé, un diamètre de maximum 15 mm et/ou une longueur de 30 à 50 mm ou de 70 à 100 mm.

8. Stent selon l'une des revendications 1 à 7,
**caractérisé par** le fait
l'extrémité distale du corps de support (2) est effilée.

9. Stent selon l'une des revendications 1 à 8,
**caractérisé par le fait**
**que** le segment de fixation (3) présente un élément de couplage (7), en particulier un couplage à billes, pour fixer le stent (1) à un élément de maintien (8) et/ou à une tige d'insertion (9).

10. Stent selon la revendication 9,
**caractérisé par le fait**
**qu'**il est prévu un élément de maintien (8), où l'élément de maintien (8) présente au moins un élément de couplage antagoniste qui peut être couplé à l'élément de couplage (7) du stent (1).

11. Système d'attelle d'une voie nasale, comportant
un stent (1) selon l'une des revendications 1 à 9 et une tige d'insertion (9) et un flexible d'insertion (13).

12. Système d'attelle d'une voie nasale selon la revendication 11,
**caractérisé par le fait**
**que** le stent peut être connecté de manière amovible à la tige d'insertion (9) et/ou le système comporte un outil de retrait (89) destiné à retirer le stent (1) d'une voie nasale, où l'outil de retrait peut être couplé au stent (1).
